# EUROPÄISCHE PATENTSCHRIFT

(11) **EP 2 815 714 B1**
(45) Veröffentlichungstag und Bekanntmachung des Hinweises auf die Patenterteilung: **31.08.2016**
(21) Anmeldenummer: 14170023.7
(22) Anmeldetag: 27.05.2014
(51) Int. Cl.: A61B 18/14, A61B 18/00

(54) **Anatomisches Ablationssystem für die Anwendung der Pulmonalvenenisolation**
Anatomical ablation system for the purpose of pulmonary vein isolation
Système d'ablation anatomique destiné à être utilisé pour l'isolation des veines pulmonaires

(30) Priorität: 18.06.2013 US 201361836172 P
(43) Veröffentlichungstag der Anmeldung: 24.12.2014
(73) Patentinhaber: VascoMed GmbH, 79589 Binzen (DE)
(72) Erfinder: Frühauf, Björn, 10365 Berlin (DE); Gellhaus, Michael, 10777 Berlin (DE); Spinelli, Daniele, 84013 Cava de Tirreni (SA) (IT)
(74) Vertreter: Galander, Marcus

(56) Entgegenhaltungen:
- WO-A1-91/11213
- US-A- 5 358 478
- US-A1- 2002 111 618
- US-A1- 2008 188 850
- US-A1- 2010 145 331

## Beschreibung

Die Erfindung betrifft eine Ablationskatheteranordnung zur Hochfrequenzablation von Gewebsabschnitten an der Wandung eines Hohlorgans oder Gefäßes eines Patienten, insbesondere zur Pulmonalvenenisolation.

Ablationskatheteranordnungen sind aus US 2008/0188850 und aus US 5358478 bekannt.

Bestimmte Herzrythmusstörungen lassen sich bekanntermaßen dadurch therapieren, dass lokal Herzgewebe zerstört wird, welches als (unkorrekter) Zeitgeber wirkt oder Teil einer Leitungsbahn für derart unerwünschte Impulse ist. Für diesen Zweck wurden verschiedene Arten von am Herzen einzusetzenden Katheteranordnungen entwickelt, die als Ablationskatheteranordnungen oder verkürzt Ablationskatheter bezeichnet werden. Die meisten dieser Anordnungen, die auch im klinischen Einsatz sind, arbeiten mit hochfrequentem (HF) Strom, der über Elektroden am Katheterende in das zu ablatierende Gewebe eingespeist wird und dieses durch die resultierende Erwärmung zerstört. Besonders bei der Therapie von Vorhofflimmern, und speziell bei jüngeren Patienten, hat sich diese Methode bewährt. Es wurden auch Verfahren und Katheteranordnungen entwickelt, die sich anderer Energiequellen bedienen, etwa Laserstrahlung, Ultraschall oder eines Kältemittels, mit dem das zu ablatierende Gewebe statt durch Erwärmung durch Unterkühlung zerstört wird. Diese Verfahren und Katheteranordnungen haben bisher aber nur geringere klinische Bedeutung im Vergleich zur HF Ablation erlangt.

Die zirkumferentielle oder lineare Pulmonalvenenisolation ist eine elektrochirurgische, minimalinvasive Methode zur Behandlung von idiopathischem oder paroxysmalem (anfallsweisem) Vorhofflimmern. Durch ein venöses Blutgefäß in der Leiste wird ein Katheter über die Hohlvene in den rechten Herzvorhof eingeführt und durch die Herzscheidewand hindurch im linken Vorhof platziert. Von dort werden mit einem Messkatheter die Pulmonalvenenwände abgetastet und im Computer eine dreidimensionale Rekonstruktion, die sogenannte Map, erstellt. Anschließend wird die vorhofnahe Muskulatur der Lungenvenen mittels Hochfrequenzstrom-Katheterablation verödet, um eine Weiterleitung der myoelektrischen Impulse auf den Vorhof zu verhindern. Im Gegensatz zur medikamentösen Therapie, bei der Empfindlichkeit der Vorhofzellen und damit die Impulsweiterleitung nur temporär reduziert wird, ist die Pulmonalvenenisolation dauerhaft. Es konnte gezeigt werden, dass Patienten mit Vorhofflimmern nach einer Katheterablation ein signifikant niedrigeres Risiko für Alzheimer-Erkrankung und Demenz als medikamentös behandelte Patienten hatten.

Der beschriebene Eingriff ist mit bekannten Katheteranordnungen zeitaufwändig und erfordert große Erfahrung des Operateurs; vielfach sind nach einigen Monaten Zweiteingriffe erforderlich, um eventuell verbliebene Lücken in den Ablationslinien zu schließen.

Der Erfindung liegt daher die Aufgabe zu Grunde, eine für die Pulmonalvenenisolation gut geeignete Ablationskatheteranordnung anzugeben, die insbesondere geringere Anforderungen an die Erfahrung des Operateurs stellt und eine Verkürzung der Eingriffszeit ermöglicht und durch deren Einsatz Zweiteingriffe und die Notwendigkeit der dreidimensionalen computerunterstützten Rekonstruktion weitgehend unnötig werden.

Diese Aufgabe wird durch eine Ablationskatheteranordnung mit den Merkmalen des Anspruchs 1 gelöst. Zweckmäßige Fortbildungen des Erfindungsgedankens sind Gegenstand der abhängigen Ansprüche.

Die Erfindung schließt den Gedanken ein, den Operateur durch eine geeignete konstruktive Ausgestaltung der Ablationskatheteranordnung bei der Einhaltung einer zweckmäßigen Ablationsspur an der Wand der Pulmonalvene zu unterstützen. Sie schließt weiter den Gedanken ein, hierzu eine Zwangsführung für das distale Katheterende vorzusehen. Weiterhin schließt die Erfindung den Gedanken des Einsatzes einer einfach zu handhabenden Vorschubsteuerung zur Fortbewegung des Katheterendes längs der gewünschten Spur ein.

Speziell bei Ablationskathetern mit einem Spülkanal, die im Interesse einer präzisen Begrenzung der Ablationspunkte durch gezielte Kühlung nach dem Wärmeeintrag als grundsätzlich vorteilhaft anzusehen sind, ist der mechanische Aufbau komplex. Die zusätzliche Integration von Bewegungsbahn-Steuermitteln in den Körper des Ablationskatheters stößt hier auf Probleme, für die die Erfindung eine besonders vorteilhafte Lösung bietet. Die Zuordnung einer außerhalb des Ablationskatheters liegenden Zwangsführung und Vorschubsteuerung vermeidet eine zusätzliche Verkomplizierung des Aufbaus des Ablationskatheters und die damit unweigerlich verbundenen Kosten- und Zuverlässigkeitsprobleme. Zudem bietet die vorgeschlagene Lösung eine höhere Flexibilität bei der Zusammenstellung von Katheteranordnungen für differenzierte Einsatzzwecke und -bedingungen.

Zur Bereitstellung einer äußerlich geschlossenen und leicht zu handhabenden Katheteranordnung können jedoch der Ablationskatheter - bei dem es sich durchaus um einen handelsüblichen Typ handeln kann - und die Führungsbahneinrichtung gemeinsam in einem langgestrecktem, flexiblen Grundkörper untergebracht sein.

In einer Ausführung der Erfindung ist die Führungsbahneinrichtung zum Führen des distalen Endes des Ablationskatheters längs einer spiralig oder helical gekrümmten Bewegungsbahn ausgebildet. Diese Ausführung entspricht weitgehend den anatomischen Gegebenheiten am hauptsächlichen Einsatzort, nämlich am Übergang des linken Vorhofs in die Pulmonalvene. Ausführungen für andere Einsatzzwecke können sich durch andersartig gekrümmte bevorzugte Bewegungsbahnen und entsprechend konfigurierte Führungsbahneinrichtungen auszeichnen, ohne dass das Konzept der Erfindung hierdurch aufgegeben würde.

In weiteren Ausführungen der Erfindung weist die Führungsbahneinrichtung einen im Gebrauchszustand räumlich gekrümmten Führungsdraht oder eine räumlich gekrümmte Führungshülse auf, auf/in der das distale Ende des Ablationskatheters beim Vorschieben und Zurückziehen gleitet. Gemäß der vorab erwähnten, aus derzeitiger Sicht bevorzugten, geometrischen Konfiguration der Bewegungsbahn ist auch der Führungsdraht oder die Führungshülse insbesondere spiralig oder helical gekrümmt, es sind jedoch für andere Einsatzbereiche auch andere geometrische Konfigurationen möglich. In einer weiteren Ausführung ist vorgesehen, dass die Führungsbahneinrichtung einen Krümmungsabschnitt aus einer Formgedächtnislegierung aufweist, der seine funktionsbestimmende Form nach dem Einführen in das Hohlorgan oder Gefäß annimmt. Diese Ausführung ermöglicht es in vorteilhafter Weise, die Führungsbahneinrichtung leicht zu nutzen und am Einsatzort ohne komplizierte Handhabungen zu konfigurieren; für ihre Konfiguration stehen aber auch andere Mittel zur Verfügung, wie etwa spezielle Einführungshülsen oder -drähte, von denen die Führungsbahneinrichtung am Einsatzort freigegeben wird und daraufhin die gewünschte Gestalt annimmt. Auch bei Einsatz eines Formgedächtnis-Materials können derartige zusätzliche Hilfsmittel zumindest zum Zurückführen der Ablationskatheteranordnung nach erfolgtem Eingriff zweckmäßig sein.

In einer weiteren Ausführung ist vorgesehen, dass der Führungsbahneinrichtung extrakorporal betätigbare Verstellmittel zur Verstellung der Bewegungsbahn zugeordnet sind. Hiermit lässt sich in vorteilhafter Weise eine Veränderung der mit der Katheterspitze zu verfolgenden Ablationsspur in Anpassung an die spezifischen anatomischen Gegebenheiten eines Patienten erreichen, ohne dass jeweils patientenspezifische Führungsbahneinrichtungen hergestellt werden müssten. Im Endeffekt ist hiermit eine Erhöhung der Erfolgswahrscheinlichkeit der Pulmonalvenenisolation, bei zugleich verringerter Wahrscheinlichkeit eines Zweiteingriffs, zu erreichen.

Hierbei kann insbesondere vorgesehen sein, dass die Verstellmittel zur Veränderung der seitlichen Erstreckung und/oder seitlichen Ablenkung einer spiraligen oder helicalen Bewegungsbahn, insbesondere eines spiralig oder helical gekrümmten Führungsdrahtes oder einer Führungshülse, ausgebildet sind. Bei diesen Ausgestaltungen geht es also weniger um eine Veränderung der Grundform der Katheter-Bewegungsbahn (und somit Ablationsspur), sondern vorrangig um die Anpassung an unterschiedliche, patientenspezifische Innenabmessungen des linken Vorhofs bzw. Eingangs der Pulmonalvene bei gleichzeitiger Gewährleistung eines hinreichenden Anpressdruckes.

In einer weiteren Ausführungsform weisen die Verstellmittel eine verstellbare Schleuse mit Schiebe-Mechanik zur lateralen Verschiebung einer Durchgangsöffnung für den Ablationskatheter bzw. den Führungsdraht oder die Führungshülse in einem Schleusenkörper auf.

In dieser Ausführung können an sich bekannte Konstruktionselemente zum Aufbau der erfindungsgemäßen Ablationskatheteranordnung genutzt und somit der Konstruktionsaufwand und die Herstellungskosten verringert werden. Speziell kann hierbei vorgesehen sein, dass die Verstellmittel eine verstellbare Schleuse mit Schiebe-Mechanik einer Mapping-Katheteranordnung aufweisen, wie sie beispielsweise von der Anmelderin unter dem Produktnamen "LEXX" hergestellt und vertrieben wird.

Vorteile und Zweckmäßigkeiten der Erfindung ergeben sich im Übrigen aus der nachfolgenden Beschreibung von Ausführungsaspekten und -beispielen der Erfindung anhand der Figuren.

Es zeigen:
- Fig. 1: eine schematische Darstellung einer Katheteranordnung gemäß einer ersten Ausführungsform der vorliegenden Erfindung und
- Fig. 2: eine schematische Darstellung einer Katheteranordnung gemäß einer zweiten Ausführungsform der vorliegenden Erfindung.

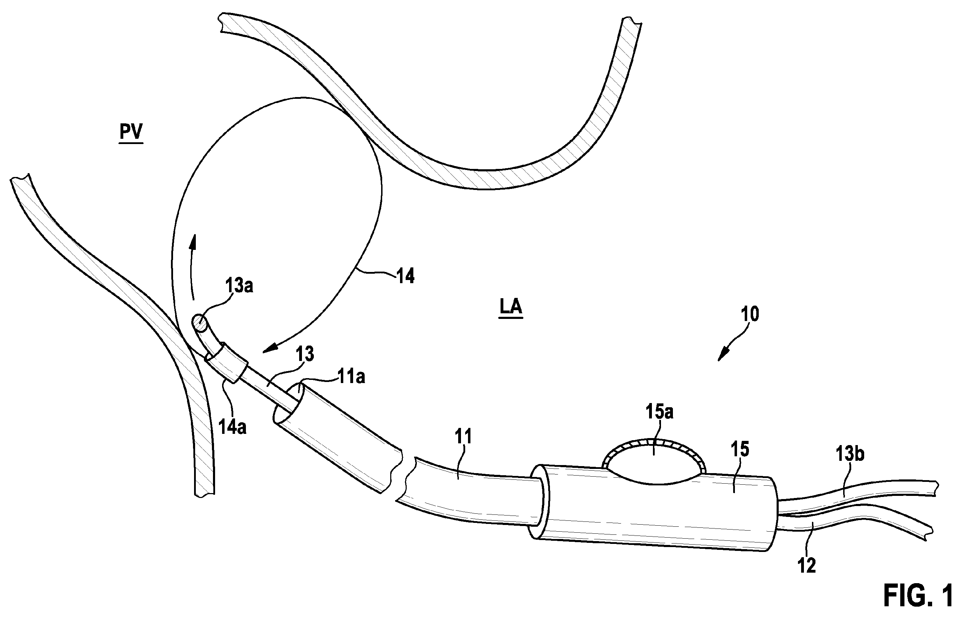

Fig. 1 zeigt skizzenartig eine Katheteranordnung 10 zur Pulmonalvenenisolation, d.h. zur Ablation von Leitungsbahnen in der Wand der aus dem linken Vorhof LA abgehenden Pulmonalvene PV. Die Ablationskatheteranordnung 10 umfasst einen langgestreckten, biegsamen Katheterkörper 11 mit einem (nicht dargestellten) Spülkanal, mit dem am proximalen Katheterende eine Spülflüssigkeits-Zuleitung 12 verbunden ist, und einem Ablationskatheter 13, der am distalen Ende eine Ablationselektrode 13a und am proximalen Ende eine Elektrodenzuleitung 13b aufweist.

In der vereinfachten Darstellung ist das distale Ende 11a des Katheterkörpers 11 zugleich als Spülöffnung zum Spülen des Behandlungsortes am Abgang der Pulmonalvene PV zu verstehen. Aus dem distalen Ende 11a des Katheterkörpers 11 ragt ein annähernd kreisförmig gekrümmter Ablations-Führungsdraht 14 aus dem Katheterkörper derart heraus, dass er sich längs des gesamten Umfangs an die Innenwand der Pulmonalvene PV anlegt.

Das distale Ende des Ablationskatheters 13 mit der Ablationselektrode 13a ist mittels eines Gleitstücks 14a gleitbar mit dem Ablations-Führungsdraht 14 verbunden und gleitet bei einem Vorschub, der über ein Vorschub-Steuerrad 15a an einer Handhabe 15 am proximalen Ende der Ablationskatheteranordnung 10 bewirkt wird, längs des Ablations-Führungsdrahtes 14 vorwärts.

Hierdurch wird das distale Ende des Ablationskatheters 13 zwangsweise längs einer annähernd kreisförmigen bis leicht helicalen räumlichen Bewegungsbahn entlang der Wand des Ostiums bzw. Antrums geführt. Nach jeweils einer kleinen Vorschubstrecke, die über das Stellrad 15a präzise eingestellt werden kann, erreicht die Ablationselektrode 13a somit zuverlässig einen nächsten Punkt an der Venenwandung, an dem ein Ablationsimpuls gesetzt und hiermit präzise ein weiterer Punkt der Wandung verödet werden kann. Üblicherweise wird dies beim Vorschieben des Ablationskatheters 13 aus dem Katheterkörper 11 heraus erfolgen, grundsätzlich ist es aber auch möglich, das distale Ende des Ablationskatheters zunächst bis zum Ende des Ablations-Führungsdrahtes 14 vorzuschieben und die sequenzielle Ablation jeweils benachbarter Wandungsbereiche beim Zurückziehen vorzunehmen.

Der Ablations-Führungsdraht 14 weist bevorzugt eine gewisse Federelastizität auf, so dass er sich beim Herausschieben aus dem Katheterkörper 11 elastisch an die Organ-Wandung in der jeweiligen anatomischen Konfiguration anlegt. Er kann auch aus einem Gedächtnismaterial bestehen, so dass er die gezeigte und hinsichtlich ihrer Funktion beschriebene Form erst an Ort und Stelle bei Körpertemperatur annimmt. Neben der gezeigten sind auch andere vorgeprägte Formen des Ablations-Führungsdrahtes sinnvoll realisierbar, insbesondere Mischformen aus einer helicalen und spiraligen Krümmung, die vorteilhaft an die anatomischen Gegebenheiten am Übergang zwischen linkem Vorhof und Pulmonalvene (oder bei Anpassung an ein anderes Hohlorgan an die anatomischen Gegebenheiten in jenem Organ) angepasst sind.

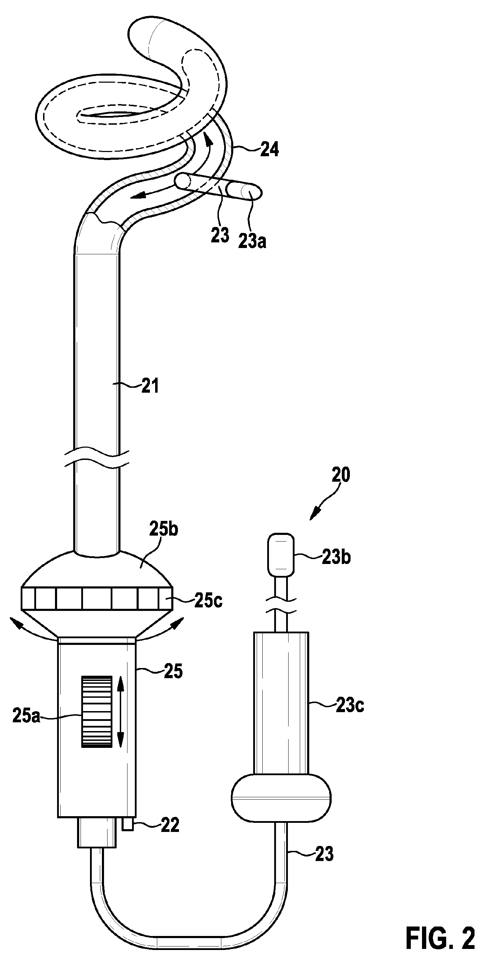

Fig. 2 zeigt als weiteres Ausführungsbeispiel schematisch eine Ablationskatheteranordnung 20, deren Aufbau teilweise ähnlich zur Ablationskatheteranordnung 10 nach Fig. 1 ist. Gleiche oder funktionsgleiche Teile sind daher mit an Fig. 1 angelehnten Bezugsziffern bezeichnet und werden nachfolgend nicht nochmals beschrieben.

Eine wesentliche Abweichung besteht darin, dass bei der Ablationskatheteranordnung 20 ein distaler Endabschnitt des Katheterkörpers 21 zugleich als (verstellbare) Führungshülse 24 für das distale Ende des Ablationskatheters 23 ausgebildet ist. Wie in der Figur zu erkennen ist, ragt das distale Ende des Ablationskatheters und somit die Ablationselektrode 23a an einer einstellbaren Position aus der Wandung der Führungshülse 24 zur Vorgabe der Bewegungsbahn der Ablationselektrode heraus. Somit können wiederum Ablationsimpulse an wählbaren Punkten einer vorbestimmten Bewegungsbahn gesetzt und somit Verödungsbereiche in einem Hohlorgan (speziell etwa der Pulmonalvene) präzise gesetzt werden.

Der Vorschub des Ablationskatheters längs der vorgegebenen Bewegungsbahn erfolgt wieder mittels eines Stellrädchens 25a als Vorschubsteuermittel, wobei bei der hier gezeigten Ausführung eine zusätzliche Handhabe 23c am proximalen Ende des Ablationskatheters 23 vorgesehen ist, durch die ebenfalls ein Vorschub des Ablationskatheters erfolgen kann.

Die Handhabe 25 umfasst hier eine verstellbare Schleuseneinrichtung 25b mit einem zweiten Stellrad 25c, womit sich die geometrische Konfiguration des Katheterendes, also der Führungshülse 24, in Anpassung an die anatomischen Gegebenheiten eines speziellen Patienten voreinstellen lässt. Anders als bei der Ausführung nach Fig. 1, wo sich der Ablations-Führungsdraht mittels seiner Federelastizität selbsttätig an die Wandung des Hohlorgans (der Pulmonalvene o.ä.) anlegt, geschieht eine entsprechende Einstellung, also bei der Ablationskatheteranordnung 20 nach Fig. 2, manuell durch den Operateur. Dies kann bei problematischen anatomischen Gegebenheiten vorteilhaft sein.

Die Funktionsweise der zugehörigen Verstelleinrichtung kann als an sich bekannt vorausgesetzt werden; einfache Verstellmechanismen mit ähnlicher Funktion sind etwa aus US 4,960,134 oder US 4,777,955 bekannt.

Die Ausführung der Erfindung ist nicht auf dieses Beispiel und die oben hervorgehobenen Aspekte beschränkt, sondern ebenso in einer Vielzahl von Abwandlungen möglich, die im Rahmen fachgemäßen Handelns liegen.

## Patentansprüche

1. Ablationskatheteranordnung (10; 20) zur Hochfrequenzablation von Gewebsabschnitten an der Wandung eines Hohlorgans (PV, LA) oder Gefäßes eines Patienten, insbesondere zur Pulmonalvenenisolation, aufweisend einen langgestreckten biegsamen Katheterkörper (11; 21) mit einem Ablationskatheter (13; 23) und einer Elektrodenzuleitung (13b) und einem Spülkanal im Katheterkörper sowie einer Ablationselektrode (13a; 23a) und einer Spülöffnung (11a) an einem distalen Ende des Katheterkörpers, Vorschubsteuermittel zum Vorschieben oder Zurückziehen des distalen Endes des Ablationkatheters relativ zur Wandung des Hohlorgans oder Gefäßes, und eine Führungshülse (24) zum zwangsweisen Führen des distalen Endes des Ablationskatheters längs einer gekrümmten räumlichen Bewegungsbahn beim Vorschieben und Zurückziehen, wobei der Führungshülse (24) extrakorporal betätigbare Verstellmittel (25b, 25c) zur Verstellung der Bewegungsbahn zugeordnet sind,
**dadurch gekennzeichnet, dass** die Verstellmittel (25b, 25c) eine verstellbare Schleuse (25b) mit Schiebe-Mechanik zur lateralen Verschiebung einer Durchgangsöffnung für die Führungshülse in einem Schleusenkörper (25b) aufweisen.

2. Ablationskatheteranordnung nach Anspruch 1, wobei die Führungshülse (24) außerhalb des Ablationskatheters (13, 23) und von diesem lösbar angeordnet ist.

3. Ablationskatheteranordnung nach Anspruch 1 oder 2, wobei die Führungshülse (24) zum Führen des distalen Endes des Ablationskatheters (13; 23) längs einer spiralig oder helical gekrümmten Bewegungsbahn ausgebildet ist.

4. Ablationskatheteranordnung nach einem der vorangehenden Ansprüche, wobei der Ablationskatheter (13', 23) und die Führungshülse (24) gemeinsam mindestens zu einem wesentlichen Teil im Katheterkörper (11', 21) aufgenommen sind.

5. Ablationskatheteranordnung nach einem der vorangehenden Ansprüche, wobei die Führungshülse (24) als eine räumlich, insbesondere kreisförmig, spiralig oder helical, gekrümmte Führungshülse (24) ausgeführt ist, auf/in der das distale Ende des Ablationskatheters (13; 33) beim Vorschieben und Zurückziehen gleitet.

6. Ablationskatheteranordnung nach einem der vorangehenden Ansprüche, wobei die Führungsbahneinrichtung (24) einen Krümmungsabschnitt aus einer Formgedächtnislegierung aufweist, der seine funktionsbestimmende Form nach dem Einführen in das Hohlorgan (PV, LA) oder Gefäß annimmt.

7. Ablationskatheteranordnung nach einem der vorangehenden Ansprüche , wobei die Verstellmittel (25b, 25c) zur Veränderung der seitlichen Erstreckung einer kreisförmigen, spiraligen oder helicalen Bewegungsbahn, einer Führungshülse (24), ausgebildet sind.

## Claims

1. An ablation catheter arrangement (10; 20) for high-frequency ablation of tissue portions on the wall of a hollow organ (PV, LA) or vessel of a patient, in particular for pulmonary vein isolation, said ablation catheter arrangement comprising an elongate flexible catheter body (11; 21) having an ablation catheter (13; 23) and an electrode feed line (13b) and a rinse duct in the catheter body, and also an ablation electrode (13a; 23 a) and a rinse opening (11a) at a distal end of the catheter body, an advance control means for advancing or withdrawing the distal end of the ablation catheter relative to the wall of the hollow organ or vessel, and
a guide sleeve (24) for forcibly guiding the distal end of the ablation catheter along a curved three-dimensional movement path during advance and withdrawal, wherein the guide sleeve (24) is assigned adjustment means (25b, 25c) that are actuatable extracorporeally in order to adjust the movement path,
**characterised in that** the adjustment means (25b, 25c) comprise an adjustable port (25b) with slide mechanism for laterally displacing a through-opening for the guide sleeve in a port body (25b).

2. The ablation catheter arrangement according to claim 1, wherein the guide sleeve (24) is arranged outside the ablation catheter (13, 23) and is detachable therefrom.

3. The ablation catheter arrangement according to claim 1 or 2, wherein the guide sleeve (24) is designed to guide the distal end of the ablation catheter (13; 23) along a spirally or helically curved movement path.

4. The ablation catheter arrangement according to any one of the preceding claims, wherein the ablation catheter (13' 23) and the guide sleeve (24) are received jointly, at least to a significant extent, in the catheter body (11', 21).

5. The ablation catheter arrangement according to any one of the preceding claims, wherein the guide sleeve (24) is embodied as a three-dimensionally, in particular circularly, spirally or helically curved guide sleeve (24), over/in which the distal end of the ablation catheter (13; 33) slides during advance and withdrawal.

6. The ablation catheter arrangement according to any one of the preceding claims, wherein the guide path device (24) comprises a curvature portion formed from a shape-memory alloy, which adopts its functional shape after insertion into the hollow organ (PV, LA) or vessel.

7. The ablation catheter arrangement according to any one of the preceding claims, wherein the adjustment means (25b, 25c) are designed to change the lateral extent of a circular, spiralled or helical movement path of a guide sleeve (24).

## Revendications

1. Système d'ablation de cathéter (10; 20) pour l'ablation par haute fréquence de parties de tissu sur la paroi d'un organe creux (PV, LA) ou d'un vaisseau d'un patient, notamment pour l'isolation de veines pulmonaires, présentant un corps de cathéter (11 ; 21) allongé flexible avec un cathéter d'ablation (13 ; 23) et une ligne d'électrode (13b) et un canal de lavage dans le corps de cathéter ainsi qu'une électrode d'ablation (13a ; 23a) et un orifice pour le lavage (11a) à une extrémité distale du corps de cathéter,
des moyens de commande d'avancement pour l'avancement ou le retrait de l'extrémité distale du cathéter d'ablation par rapport à la paroi de l'organe creux ou du vaisseau, et
une gaine de guidage (24) pour le guidage forcé de l'extrémité distale du cathéter d'ablation le long d'une trajectoire de mouvement courbe dans l'espace lors de l'avancement et du retrait, où des moyens de réglage (25b, 25c) pouvant être actionnés de manière extracorporelle sont associés à la gaine de guidage (24) pour le réglage de la trajectoire de mouvement,
**caractérisé en ce que** les moyens de réglage (25b, 25c) présentent un barillet (25b) avec un mécanisme de coulissement pour le coulissement latéral d'un orifice de passage pour la gaine de guidage dans un corps de barillet (25b).

2. Système d'ablation de cathéter selon la revendication 1, où la gaine de guidage (24) est disposée en dehors du cathéter d'ablation (23, 23) et peut en être détachée.

3. Système d'ablation de cathéter selon la revendication 1, ou 2, où la gaine de guidage (24) est conçue pour le guidage de l'extrémité distale du cathéter d'ablation (13 ; 23) le long d'une trajectoire de mouvement courbe de type spirale ou hélicoïdal.

4. Système d'ablation de cathéter selon l'une des revendications précédentes, où le cathéter d'ablation (13' ; 23) et la gaine de guidage (24) sont logés ensemble au moins dans la plus grande partie dans le corps de cathéter (11', 21).

5. Système d'ablation de cathéter selon l'une des revendications précédentes, où la gaine de guidage (24) est conçue sous la forme d'une gaine de guidage courbe dans l'espace, notamment en forme de cercle, de spirale ou d'hélice,
sur/dans laquelle l'extrémité distale du cathéter d'ablation (13 ; 33) glisse lors de l'avancement et du retrait.

6. Système d'ablation de cathéter selon l'une des revendications précédentes, où le dispositif de piste de guidage (24) présente une section de courbure constituée en un alliage à mémoire de forme, qui adopte sa forme prévue pour le fonctionnement après l'insertion dans l'organe creux (PV, LA) ou dans le vaisseau.

7. Système d'ablation de cathéter selon l'une des revendications précédentes, où les moyens de réglage (25b, 25c) sont conçus pour la modification de l'allongement latéral d'une trajectoire de mouvement en forme de cercle, de spirale ou d'hélice d'une gaine de guidage (24).
